# EUROPÄISCHE PATENTSCHRIFT

(11) **EP 1 140 781 B1**
(45) Veröffentlichungstag und Bekanntmachung des Hinweises auf die Patenterteilung: **01.10.2003**
(21) Anmeldenummer: 99963453.8
(22) Anmeldetag: 09.12.1999
(51) Int. Cl.: C07C 68/08, C07C 69/96

(54) **VERFAHREN ZUR AUFARBEITUNG VON DIARYLCARBONATHALTIGEN REAKTIONSMISCHUNGEN**
METHOD FOR REPROCESSING REACTION MIXTURES CONTAINING DIARYL CARBONATE
PROCEDE PERMETTANT DE RETRAITER DES MELANGES DE REACTION RENFERMANT DES DIARYLCARBONATES

(30) Priorität: 22.12.1998 DE 19859296
(43) Veröffentlichungstag der Anmeldung: 10.10.2001
(73) Patentinhaber: Bayer Aktiengesellschaft, 51368 Leverkusen (DE)
(72) Erfinder: HESSE, Carsten, D-47918 Tönisvorst (DE); JANSEN, Ursula, D-47799 Krefeld (DE); RECHNER, Johann, D-47906 Kempen (DE); REISINGER, Claus-Peter, D-47798 Krefeld (DE); EEK, Rob, D-51061 Köln (DE); HALLENBERGER, Kaspar, D-51375 Leverkusen (DE); FRIEDRICH, Martin, D-51069 Köln (DE)
(86) Internationale Anmeldenummer: EP9909695
(87) Internationale Veröffentlichungsnummer: WO00037418

(56) Entgegenhaltungen:
- EP-A- 0 687 666
- EP-A- 0 801 052

## Beschreibung

Die vorliegende Erfindung betrifft ein Verfahren zur Aufarbeitung von Diarylcarbonat, aromatische Hydroxyverbindung, Wasser, Base und quaternäres Salz enthaltenden Reaktionsmischungen, die bei der Herstellung von Diarylcarbonaten durch Direktcarbonylierung von aromatischen Hydroxyverbindungen erhalten werden. Bei dem erfindungsgemäßen Verfahren wird einerseits aus der Reaktionsmischung ein Addukt von Diarylcarbonat und aromatischer Hydroxyverbindung kristallisiert. andererseits aus der Flüssigphase ein die aromatische Hydroxyverbindung enthaltendes Destillat entfernt. Die Flüssigphase wird anschließend dem Reaktionsschritt der Direktcarbonylierung wieder zugeführt. Durch das erfindungsgemäße Verfahren wird die Desaktivierung des im Kreis geführten Katalysatorsystems minimiert.

In US-PS 5 239 106 wird ein Verfahren zur Isolierung von Adduktkristallen aus Diphenylcarbonat (DPC) und Phenol im molaren Verhältnis von 1:1, aus phenolischen Lösungen mit einem DPC-Gehalt von 20 bis 70 Gew.-% offenbart. Bei der Herstellung des Diphenylcarbonats wird das während der Reaktion gebildete Wasser mittels Molsieb entfernt. Wegen der erforderlichen Mengen an Molsieb und der aufwendigen Regeneration des beladenen Molsiebs ist eine großtechnische Umsetzung dieses Verfahrens wirtschaftlich wenig attraktiv.

EP-A 583 938 lehrt, bei dem in US-PS 5 239 106 beschriebenen Verfahren die Mutterlauge der Kristallisation wieder in den Reaktionsschritt zurückzuführen. Wie aus Beispiel 1 und 2 der EP-A 583 938 hervorgeht, führt die Rückführung der Mutterlauge in die Reaktion zu einer starken Desaktivierung des Katalysatorsystems

Aus US-PS 5 498 742 geht ein alternatives Verfahren zur Herstellung von Diarylcarbonaten hervor, bei dem das in der Reaktion gebildete Wasser durch überschüssiger Reaktionsgas ausgestrippt wird. Wird das gebildete Diarylcarbonat durch Kristallisation abgetrennt und die Mutterlauge der Kristallisation in die Reaktionsstufe zurückgeführt, werden auch hier Ausbeute- und Selektivitätsverluste beobachtet.

EP-A-0 801 052 offenbart ein Verfahren zur Herstellung von Diarylcarbonat (DAC) durch oxydative Carbonylierung (Direktcarbonylierung). Aus dem Reaktionsgemisch wird DAC auskristallisiert und die Mutterlauge, worin der Katalysator enthalten ist, wird in den Reaktor zur Herstellung des Diarylcarbonats zurückgeführt.

Dieses Verfahren führt zu einer starken Desaktivierung des Katalysatorsystems und es führt zu Ausbeute- und Selektivitätsverlusten.

Es wurde nun gefunden, dass sich die Desaktivierung des Katalysatorsystems weitgehend vermeiden lässt, wenn vor der Rückführung von katalysatorhaltigen Lösungen in den Reaktionsschritt ein Teil der aromatischen Hydroxyverbindung destillativ entfernt wird.

Gegenstand der Erfindung ist ein Verfahren zur Aufarbeitung von Reaktionsmischungen aus der Herstellung von Diarylcarbonaten durch Direktcarbonylierung von aromatischen Hydroxyverbindungen, bei dem aus einer Diarylcarbonat, aromatische Hydroxyverbindung, Wasser, Base und quatemäres Salz enthaltenden Reaktionsmischung ein Addukt von Diarylcarbonat und aromatischer Hydroxyverbindung kristallisiert wird, aus der Flüssigphase bei Drücken von 10 bis 200 mbar und Temperaturen von 40 bis 160°C mindestens 0,2 Gew.-%, bezogen auf die Reaktionsmischung vor der Kristallisation, eines die aromatische Hydroxyverbindung enthaltenden Destillats entfernt wird, und die Flüssigphase anschließend in den Reaktionsschritt zurückgeführt wird.

Dabei kann die Kristallisation des Diphenylcarbonat-Addukts prinzipiell vor oder nach der Entfernung des die aromatische Hydroxyverbindung enthaltenden Destillats erfolgen. Im erstgenannten Fall ist darauf zu achten, dass nicht so viel aromatische Hydroxyverbindung aus der Reaktionsmischung entfernt werden darf, dass der Diarylcarbonatgehalt in der Flüssigphase über 70 Gew.-% ansteigt, da sonst nicht mehr das Diarylcarbonat-Addukt auskristallisiert, sondern reines Diarylcarbonat. Bevorzugt wird man so viel Destillat entfemen, dass in der Reaktionsmischung vor der Kristallisation 25 bis 65 Gew.-%, besonders bevorzugt 35 bis 55 Gew.-% Diarylcarbonat enthalten sind, um die Kristallisation besonders effektiv zu gestalten.

Um die Desaktivierung des Katalysatorsystems zu minimieren, müssen nach der Reaktion mindestens 0,2 Gew.-% des Reaktionsmischung als Destillat entfernt werden, bevor die Flüssigphase in den Reaktionsschritt zurückgeführt werden kann. Wenn es nicht notwendig ist, die Reaktionsmischung vor der Aufarbeitung an Diarylcarbonat anzureichern, genügt es in der Regel, eine 0,2 bis 5 Gew.-% der Reaktionsmischung entsprechende Menge Destillat zu entfernen. Führt man die Destillation nach dem Kristallisationsschritt durch, wird man bevorzugt auch nur eine 0,2 bis 5 Gew.-% der Reaktionsmischung entsprechende Menge an Destillat entfernen, um die in der Flüssigphase enthaltenen Katalysatorkomponenten einer möglichst geringen thermischen Belastung auszusetzen und den nötigen Energieaufwand gering zu hallen.

Die Herstellung von Diarylcarbonaten durch Direktcarbonylierung von aromatischen Hydroxyverbindungen ist bekannt (siehe z.B. US-A 4,349,485, US-A 5,231,210, EP-A 667 336, EP-A 858 991, US-A 5,760,272).

Dabei wird eine aromatischen Hydroxyverbindung der Formel

R-O-H (1),

worin
- R: substituiertes oder nicht substituiertes C₆-C₁₂-Aryl, bevorzugt substituiertes oder nicht substituiertes Phenyl, besonders bevorzugt nicht substituiertes Phenyl bedeutet,
mit Kohlenmonoxid und Sauerstoff in Gegenwart eines Platinmetall-Katalysators, eines Cokatalysators, eines quaternären Salzes und einer Base bei einer Temperatur von 30 bis 200°C, bevorzugt 30 bis 150°C, besonders bevorzugt 40 bis 120°C und einem Druck ven 1 bis 200 bar, bevorzugt 2 bis 100 bar, besonders bevorzugt 5 bis 50 bar umgesetzt.

Die Zusammensetzung der Reaktionsgase Kohlenmonoxid und Sauerstoff kann in weiten Konzentrationsgrenzen variiert werden, es wird jedoch zweckmäßigerweise ein CO:O₂-Molverhältnis (normiert auf CO) von 1:(0,001-1,0) bevorzugt 1:(0,01-0,5) und besonders bevorzugt von 1:(0,02-0,3) eingestellt. Der Sauerstoffpartialdruck ist bei diesen Molverhältnissen groß genug, um hohe Raum-Zeit-Ausbeuten erreichen zu können und gleichzeitig keine explosionsfähigen Kohlenmonoxid/Sauerstoff-Gasgemische bilden zu können. Die Reaktionsgase unterliegen keinen besonderen Reinheitsanforderungen. So kann Synthesegas als CO-Quelle und Luft als O₂-Träger dienen, es ist jedoch darauf zu achten, daß keine Katalysatorgifte wie z.B.: Schwefel oder dessen Verbindungen eingetragen werden. Bevorzugt werden reines CÖ und reiner Sauerstoff verwendet.

Bei den umsetzbaren aromatischen Hydroxyverbindungen handelt es sich beispielsweise um Phenol, o-, m- oder p-Kresol, o-, m- oder p-Chtorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol und Bisphenol A, bevorzugt um Phenol. Allgemein handelt es sich im Falle einer Substitution der aromatischen Hydroxyverbindung um 1 oder 2 Substituenten in der Bedeutung von C₁-C₄-Alkyl, C₁-C₄-Alkoxy, Fluor, Chlor oder Brom.

Einsetzbare Basen sind Alkali-, quatemäre Ammonium-, oder quatemäre Phosphoniumsalze von aromatischen Hydroxyverbindungen der Formel (I). Altemativ können auch Trialkylamine wie Tributylamin, Diisopropylethylamin, DBU, DBN oder auch andere Basen, z.B. Kalium-tert.-butanolat, verwendet werden.

Die Base wird in einer von der Stöchiometrie unabhängigen Menge zugesetzt. Das Verhältnis von Platinmetall, z.B. Palladium, zu Base wird vorzugsweise so gewählt, daß pro Grammatom Platinmetall, z.B. Palladium, 0,1 bis 500, bevorzugt 0,3 bis 200 besonders bevorzugt 0,9 bis 130 Äquivalente Base eingesetzt werden.

Das Verfahren wird vorzugsweise ohne Lösungsmittel durchgeführt. Selbstverständlich können auch inerte Lösungsmittel verwendet werden. Als Beispiele für Lösungsmittel seien Dimethylacetamid, N-Methylpyrrolidinon, t-Butanol, Cumylalkohol, Isoamylalkohol, Tetramethylhamstoff, Diethylenglykol, halogenierte Kohlenwasserstoffe (z.B. Chlorbenzol oder Dichlorbenzol) und Ether, wie Dioxan, Tetrahydrofuran, t-Butylmethylether und veretherte Glykole, genannt.

Geeignete Platinmetall-Katalysatoren bestehen aus mindestens einem Edelmetall der Gruppe VIII, vorzugsweise Palladium. Es kann in verschiedener Form zugegeben werden. Palladium kann in metallischer Form oder bevorzugt in Form von PalladiumVerbindungen der Oxidationsstufen 0 und +2, wie beispielsweise Palladium(II)-acetylacetonat, -halogenide, -carboxylate von C₂-C₆-Carbonsäuren, -nitrat, -oxide oder Palladiumkomplexe, die beispielsweise Olefine, Amine, Phosphine und Halogenide enthalten können, eingesetzt werden. Besonders bevorzugt sind Palladiumbromid und Palladiumacetylacetonat.

Die Menge an Platinmetall-Katalysator ist nicht beschränkt. Meist wird so viel Katalysator zugesetzt, daß die Konzentration des Metalls im Reaktionsansatz 1 - 3000 ppm beträgt, bevorzugt sind Konzentrationen von 5 - 500 ppm.

Als Cokatalysator wird ein Metall der Gruppen III A, III B, IV A, IV B, V B, I B, II B, VI B, VII B, der Seltenerdmetalle (Atomnummern 58-71) oder der Eisengruppe des Periodensystems der Elemente (Mendelejew) eingesetzt, wobei das Metall in verschiedenen Oxidationsstufen eingesetzt werden kann. Bevorzugt werden Mn, Cu, Co, V, Zn, Ce und Mo eingesetzt, z.B. Mangan(II), Mangan(III), Kupfer(I), Kupfer(II), Kobalt(II), Kobalt(III), Vanadium(III) und Vanadium(IV). Die Metalle können beispielsweise als Halogenide, Oxide, Carboxylate von C₂-C₆-Carbonsäuren, Diketonate oder Nitrate sowie als Komplexverbindungen, die beispielsweise Kohlenmonoxid, Olefine, Amine, Phosphine und Halogenide enthalten können, eingesetzt werden. Besonders bevorzugt werden Mn, Cu, Mo und Ce eingesetzt. Ganz besonders bevorzugt werden Manganverbindungen im erfindungsgemäßen Verfahren verwendet, besonders bevorzugt Mangan(II)komplexe, ganz besonders bevorzugt Mangan(II)acetylacetonat bzw. Mangan(III)-acetylacetonat.

Der Cokatalysator wird in einer solchen Menge zugesetzt, daß seine Konzentration im Bereich von 0,0001 bis 20 Gew.-% des Reaktionsgemisches liegt, bevorzugt ist der Konzentrationsbereich von 0,005 bis 5 Gew.-%, besonders bevorzugt 0,01 bis 2 Gew.-%.

Bei den quatemären Salzen kann es sich beispielsweise um mit organischen Resten substituierte Ammonium-, Guanidinium-, Phosphonium- oder Sulfoniumsalze handeln. Geeignet sind Ammonium-, Guanidinium-, Phosphonium- und Sulfoniumsalze, die als organische Reste C₆- bis C₁₀-Aryl-, C₇- bis C₁₂-Aralkyl- und/oder C₁- bis C₂₀-Alkyl-Reste und als Anion ein Halogenid, Tetrafluoroborat oder Hexafluorophosphat tragen. Bevorzugt werden Ammoniumsalze eingesetzt, die als organische Reste C₆- bis C₁₀-Aryl-, C₇- bis C₁₂-Aralkyl- und/oder C₁- bis C₂₀-Alkyl-Reste und als Anion ein Halogenid tragen, besonders bevorzugt ist Tetrabutylammoniumbromid. Die Menge eines solchen quaternären Salzes kann beispielsweise 0,1 - 20 Gew.-%, bezogen auf das Gewicht des Reaktionsgemisches, betragen. Vorzugsweise beträgt diese Menge 0,5 - 15 Gew.-%, besonders bevorzugt 1 - 5 Gew.-%.

Für die Diarylcarbonatherstellung können homogene Katalysatorsysteme eingesetzt - werden oder heterogene Katalysatoren, bei denen das Platinmetall oder das Platinmetall und der Cokatalysator auf einem heterogenen Träger aufgebracht sind. Bei den heterogenen Katalysatorsystemen sind die übrigen Komponenten des Katalysatorsystems, wie die Base, die quatemäre Verbindung und gegebenenfalls der Cokatalysator, weiterhin in der Reaktionslösung homogen gelöst.

Der heterogene Trägerkatalysator kann ortsfest in Rührbehältern, Blasensäulen, einem Rieselphasenreaktor oder Kaskaden dieser Reaktoren eingesetzt werden. Eine Abtrennung des Trägerkatalysators aus dem Reaktionsgemisch entfällt dann völlig.

Bei der Herstellung von Diarylcarbonaten durch Direktcarbonylierung von aromatischen Hydroxyverbindungen wird eine Diarylcarbonat, aromatische Hydroxyverbindung, Wasser, Base und quatemäres Salz enthaltende Reaktionsmischung erhalten. Wird ein homogenes Katalysatorsystem verwendet, so enthält die Reaktionsmischung außerdem noch Platinmetallkatalysator und Cokatalysator.

Die Entfernung des die aromatische Hydroxyverbindung enthaltenden Destillats aus der Reaktionsmischung wird bei Drücken von 10 bis 200 mbar und Temperaturen von 40 bis 160°C, bevorzugt 50 bis 120°C, besonders bevorzugt 60 bis 100°C durchgeführt.

Die Kristallisation der Flüssigphase kann durch verschiedene dem Fachmann geläufige Kristallisationsverfahren erfolgen, beispielsweise fraktionierende Schmelzkristallisation, Schichtkristallisation oder statische oder dynamische Suspensionskristallisation. Bevorzugt erfolgt die Kristallisation der Flüssigphase durch Suspensionskristallisation.

Nach Kristallisation und Entfernung des Destillats wird die Flüssigphase, gegebenenfalls nach Abtrennung desaktivierter Katalysatorbestandteile durch Filtration, ohne weitere Aufarbeitung dem Reaktionsschritt der Direktcarbonylierung wieder zugeführt.

Bevorzugt werden die Diarylcarbonatadduktkristalle nach der Abtrennung von der Mutterlauge zur Entfernung anhaftender Verunreinigungen, beispielsweise von Katalysatorrückständen, gewaschen. Das Waschen kann beispielsweise mit Wasser oder aromatischer Hydroxyverbindung durchgeführt werden. Bevorzugt werden die Kristalle jedoch mit einer Lösung von Diarylcarbonat in aromatischer Hydroxyverbindung gewaschen, da sich dadurch Ausbeuteverluste minimieren lassen. Bevorzugt beträgt die Diarylcarbonatkonzentration der Waschlösung 10 bis 25 Gew.-%.

Die Waschlösung kann anschließend der dem Reaktionsschritt der Direktcarbonylierung erhaltenen Reaktionsmischung zugeführt werden. Dadurch läßt sich das in der Waschlösung enthaltene Diarylcarbonat zurückgewinnen.

### Beispiele

### Beispiel 1: (Vergleichsbeispiel)

In einen Autoklaven wurden bei ca. 85°C und 14 bar über zwei Dosierpumpen pro Stunde 1200 g einer Reaktionslösung bestehend aus ca. 120 ppm Pd (als PdBr₂), ca. 300 ppm Mn (als Mn(acac)₂), ca. 2,5 Gew.-% TBAB (Tetrabutylammoniumbromid), ca. 1,5 Gew.-% TBAP (Tetrabutylammoniumphenolat), 20 Gew.-% Diphenylcarbonat und Phenol eindosiert. Der Flüssigkeitsstand im Reaktor wurde auf ca. 1200 ml eingestellt. Gleichzeitig wurde in den Autoklaven ca. 700 NL/h eines Gasgemisches bestehend aus 2.5 Vol.-% Sauerstoff, 1,5 Vol.-% Inertgas (N₂, Ar usw.) und Rest zu 100 % Kohlenmonoxid eindosiert.

Der Flüssigkeitsstrom aus dem Reaktor wurde kontinuierlich entnommen und einem Rohrbündelkristaller zugeführt; durch den getakteten Einsatz mehrerer Kristaller konnte eine quasi-kontinuierliche Fahrweise realisiert werden. Der DPC-Gehalt der Reaktionsmischung betrug ca. 30 Gew.-%. Die Impftemperatur und die Kristallisationstemperatur betrugen 39°C, die Kühlendtemperatur 20°C, die Kühlrate 3 K/h. Nach Ablassen der Mutterlauge wurde das Kristallisat durch teilweises Abschmelzen gereinigt. Die Mutterlauge und die Schwitzlösung wurden gemeinsam nach Filtration ohne weitere Aufarbeitung über die dritte Dosierpumpe dem Reaktor wieder zugeführt.

Gleichzeitig wurden die Fördermengen der ersten beiden Dosierpumpen und die Konzentrationen der Katalysatorkomponenten so eingestellt, daß die Konzentrationen und Mengenströme des Reaktorzulaufs den oben angegebenen Werten entsprach. Die Katalysatorergänzungen entsprachen den abfiltrierten Mengen. Im weiteren Verlauf wurden Mutterlauge und Schwitzlösung ständig wie oben beschrieben der Reaktion wieder zugeführt. Die entnommenen DPC- und PhOH-Mengen wurden dem System in Form von Frischphenol, aufgeteilt auf die ersten beiden Dosierungen, wieder zugeführt.

Die Aufteilung zwischen Kristallisat und Summe der Rückführlösungen betrug ca. 1 : 5,3. Das Kristallisat bestand zu 62 Gew.-% aus DPC, zu 37,5 Gew.-% aus Phenol un enthielt Spuren von Katalysatorkomponenten und Nebenprodukten.

Schon nach der ersten Rückführung sank die Raum-Zeit-Ausbeute der Reaktion von ca. 98 g/lh DPC auf 33 g/lh ab. In den Kristallern konnte zunächst kein Kristallisat gewonnen werden. Nachdem sich in der Apparatur ein Gleichgewicht eingestellt hatte, ließen sich, der Raum-Zeit-Ausbeute entsprechend, geringe Mengen Kristallisat gewinnen.

### Beispiel 2

Beispiel 1 wurde wiederholt, aber mit dem Unterschied, daß die vereinigte Rückführlösung, d.h. Mutterlauge und Schwitzlösung, nach der Filtration einem destillativen Reinigungsschritt in einem Dünnschichtverdampfer bei 70°C und 60 mbar unterworfen wurde. Bei dieser Destillation wurden von der Mutterlauge ca. 40 g/h (3.3 Gew.-%) Phenol und Leichtsieder abgetrennt und die Rückführlösung anschließend wie im Beispiel 1 beschrieben der Reaktion zugeführt.

Bis sich in der Apparatur ein Gleichgewicht eingestellt hatte, sank die Raum-Zeit-Ausbeute von ca. 96 g/lh auf 81 g/lh ab. In den Kristallern konnte ständig die der Raum-Zeit-Ausbeute entsprechende DPC-Menge in Form von DPC/PhOH-Kristallisat entnommen werden.

### Beispiel 3

Beispiel 1 wurde wiederholt, aber mit dem Unterschied, daß der DPC-Gehalt im Reaktorzulauf ca. 10 Gew.-% betrug. Nach der Reaktion betrug der DPC-Gehalt ca. 20 Gew.-% und die Raum-Zeit-Ausbeute an DPC etwa 99 g/lh. Bevor die Reaktionslösung in die Kristaller gelangte, wurde sie mit Hilfe eines Dünnschichtverdampfers bei 100°C und ca. 20 mbar von Leichtsiedern und Phenol befreit, so daß der DPC-Gehalt auf ca. 33 Gew.-% anstieg. Hierzu mußte etwa ein Drittel der ursprünglichen Lösung destillativ entfernt werden.

Die anschließende Kristallisation erfolgte wie in Beispiel 1 beschrieben. Die Aufteilung zwischen Kristallisat und Summe der Rückführlösungen betrug ca. 1 : 5,4. Das Kristallisat bestand zu 61 Gew.-% aus DPC, zu 38,5 Gew.-% Phenol und enthielt Spuren an Katalysatorkomponenten und Nebenprodukten. Die Rückführung der vereinigten Mutterlaugen und Schwitzlösungen sowie die Dosierung der Katalysatorergänzung über die Dosierpumpen 1 und 2 erfolgte wie in Beispiel 1 beschrieben.

Nachdem sich in der Apparatur ein Gleichgewicht eingestellt hatte, betrug die Raum-Zeit-Ausbeute noch ca. 79 g/lh. In den Kristallern konnte ständig die der Raum-Zeit-Ausbeute entsprechende DPC-Menge in Form von DPC/PhOH-Kristallisat entnommen werden.

## Patentansprüche

1. Verfahren zur Aufarbeitung von Reaktionsmischungen aus der Herstellung von Diarylcarbonaten durch Direktcarbonylierung von aromatischen Hydroxyverbindungen, **dadurch gekennzeichnet, dass** aus einer Diarylcarbonat, aromatische Hydroxyverbindung, Wasser, Base und quaternäres Salz enthaltenden Reaktionsmischung ein Addukt von Diarylcarbonat und aromatischer Hydroxyverbindung kristallisiert wird, aus der Flüssigphase bei Drücken von 10 bis 200 mbar und Temperaturen von 40 bis 160°C mindestens 0,2 Gew.-%, bezogen auf die Reaktionsmischung vor der Kristallisation, eines die aromatische Hydroxyverbindung enthaltenden Destillats entfernt wird, und die Flüssigphase anschließend ohne weitere Aufarbeitung dem Reaktionsschritt der Direktcarbonylierung wieder zugeführt wird.

2. Verfahren gemäß Anspruch 1, bei dem zunächst die Kristallisation des Diarylcarbonatadduktes durchgeführt wird und anschließend eine 0,2 bis 5 Gew.-% der ursprünglichen Reaktionsmischung entsprechende Menge an die aromatische Hydroxyverbindung enthaltendem Destillat entfernt wird.

3. Verfahren gemäß Anspruch 1, bei dem aus der Reaktionsmischung vor der Kristallisation so viel Destillat entfernt wird, dass deren, Diarylcarbonat-Gehalt 25 bis 65 Gew.-% beträgt und anschließend die Kristallisation des Diarylcarbonatadduktes durchgeführt wird.

4. Verfahren gemäß Anspruch 1, bei dem die Flüssigphase anschließend an die Entfernung des Destillats nach Abtrennung desaktivierter Katalysatorbestandteile ohne weitere Aufarbeitung dem Reaktionsschritt der Direktcarbonylierung wieder zugeführt wird.

## Claims

1. Process for reprocessing reaction mixtures from the production of diaryl carbonates by the direct carbonylation of aromatic hydroxy compounds, **characterised in that** an adduct of diaryl carbonate and aromatic hydroxy compound is crystallised out of a reaction mixture containing diaryl carbonate, aromatic hydroxy compound, water, base and quaternary salt, at least 0.2 wt %, referred to the reaction mixture prior to the crystallisation, of a distillate containing the aromatic hydroxy compound is removed from the liquid phase at pressures of 10 to 200 mbar and temperatures of 40 to 160 °C, and the liquid phase is then fed to the reaction step of direct carbonylation again without further reprocessing.

2. Process according to claim 1, in which first of all the crystallisation of the diaryl carbonate adduct is carried out and then an amount of distillate containing the aromatic hydroxy compound is removed, said amount corresponding to 0.2 to 5 wt % of the original reaction mixture.

3. Process according to claim 1, in which distillate is removed from the reaction mixture prior to the crystallisation in an amount such that the diaryl carbonate content of the latter comes to 25 to 65 wt %, and the crystallisation of the diaryl carbonate adduct is then carried out.

4. Process according to claim 1, in which the liquid phase is following the removal of the distillate, after the separation of deactivated catalyst components, fed to the reaction step of direct carbonylation again without further reprocessing.

## Revendications

1. Procédé de traitement de mélanges réactionnels provenant de la préparation de carbonates de diaryle par carbonylation directe de composés hydroxylés aromatiques, **caractérisé en ce que** l'on cristallise d'un mélange réactionnel contenant un carbonate de diaryle, un composé hydroxylé aromatique, de l'eau, une base et un sel quaternaire, un adduit du carbonate de diaryle et du composé hydroxylé aromatique, et on élimine de la phase liquide, à des pressions allant de 10 à 200 mbar et des températures allant de 40 à 160°C, au moins 0,2% en poids, sur base du mélange réactionnel avant la cristallisation, un distillat contenant le composé hydroxylé aromatique, et la phase liquide est ensuite ramenée sans autre traitement, dans l'étape de réaction de la carbonylation directe.

2. Procédé suivant la revendication 1, dans lequel on réalise d'abord la cristallisation de l'adduit de carbonate de diaryle et ensuite, on élimine une quantité correspondant à 0,2 à 5% en poids du mélange réactionnel initial, de distillat contenant le composé hydroxylé aromatique.

3. Procédé suivant la revendication 1, dans lequel on élimine du mélange réactionnel avant la cristallisation, assez de distillat pour que la teneur en carbonate de diaryle se situe dans l'intervalle allant de 25 à 65% en poids et ensuite, on réalise la cristallisation de l'adduit de carbonate de diaryle.

4. Procédé suivant la revendication 1, dans lequel la phase liquide, après élimination du distillat, après séparation des constituants désactivés de catalyseur, est ramenée sans autre traitement, dans l'étape de réaction de la carbonylation directe.
